# EUROPEAN PATENT SPECIFICATION

(11) **EP 2 116 178 B1**
(45) Date of publication and mention of the grant of the patent: **04.07.2012**
(21) Application number: 08425324.4
(22) Date of filing: 09.05.2008
(51) Int. Cl.: A61B 1/32

(54) **Medical device for anorectal diagnostic analyses**
Medizinische Vorrichtung für anorektale Diagnoseanalysen
Dispositif médical pour analyses de diagnostic anorectal

(43) Date of publication of application: 11.11.2009
(73) Proprietor: Sapi Med S.p.A., 15100 Alessandria (IT)
(72) Inventor: Meinero, Piercarlo, Sapi Med S.p.A., 15100 Alessandria (IT)
(74) Representative: Long, Giorgio

(56) References cited:
- WO-A-93/04727
- WO-A-94/23787
- WO-A-2006/033122
- GB-A- 1 134 972
- US-A1- 2005 277 811
- US-A1- 2007 043 264

## Description

. The present invention relates to a medical device for anorectal diagnostic analyses and, in particular, it relates to a multifunctional anoscope.

. In the proctological medical practice, devices termed anoscopes or rectoscopes are known which are adapted to allow inspection and operations directed on the anus and rectum by the practitioner. Currently, a proctologic examination involves certain procedures, ranging from anamnesis to instrumental investigations, which can be carried out at the practitioner's office (inspection, rectal exploration, anoscopy and/or rectoscopy). These common manoeuvres can identify possible diseases affecting the anus-rectum, though only some of which can be carried out by means of a conventional anoscope. As a result, to date there is no possibility to test rectal sensitivity by testing the functionality of that anatomical tract during the first examination. In fact, these valuable additional data can be obtained only by performing an anorectal manometry, an examination which requires the acquisition of a sophisticated, expensive equipment, and which is not anyhow included in the routinary examinations, moreover for more common diseases, such as haemorrhoids and mucosal prolapse of the rectum.

. Vice versa, being able to known, already during the first examination, some functional parameters of the anus-rectum would be extremely important just in order to properly individuate the diagnostic pathway for the patient. In particular, an early detection of sensitivity modifications would allow the specialist assessing the opportuneness to perform further diagnostic controls instead of put hastily the patient in for a possibly unsuitable or unnecessary surgery. US 2005/277811 A1 and WO 2006/033122 disclose devices according to the preamble of claim 1.

. Therefore, the object of the present invention is to provide a medical device which allows obtaining early, and already during the first proctologic examination, some crucial pieces of information for the diagnosis of an anorectal disease.

. Such object is achieved by a medical device as set forth in the annexed claims, the definitions of which are an integral part of the present description.

. Further characteristics, and the advantages of the present invention will be more clearly understood by the following description of an embodiment, given by way of non-limiting example only, in which:
- Fig. 1 represents a sectional side view of the medical device of the invention in an assembled form;
- Fig. 2 represents a sectional longitudinal view of a member of the device in Fig. 1;

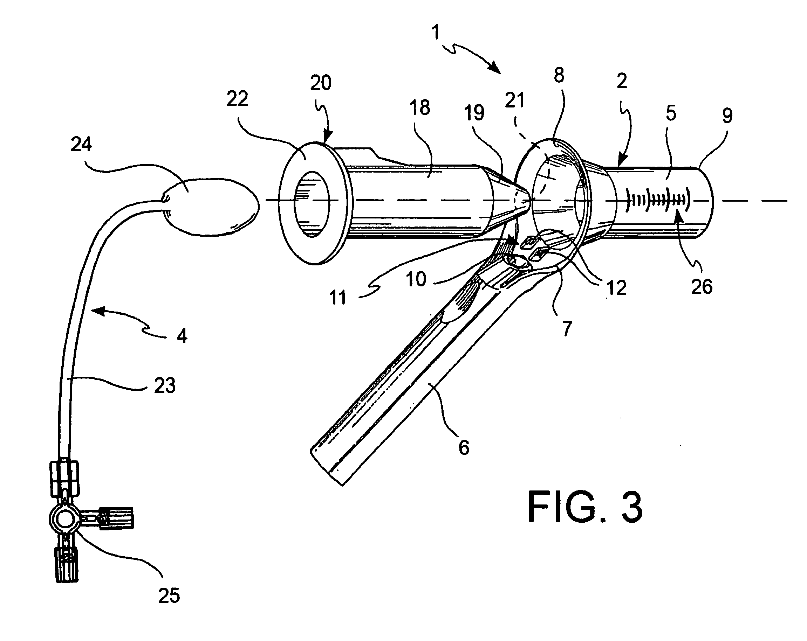

- Fig. 3 represents an exploded, perspective view of the medical device of the invention;

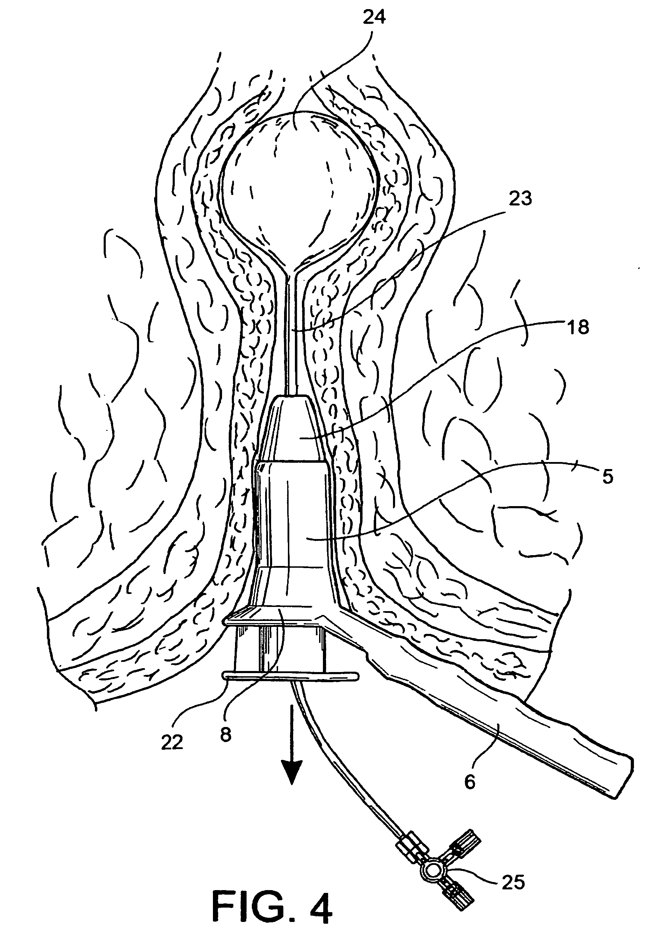

- Fig. 4 represents a schematic view of the medical device in Fig. 1 upon use;
- Fig. 5 represents a perspective view in phantom of a detail of the medical device of the invention.

. In the Figures, a medical device for proctologic inspections/operations according to the present invention is generally designated with the numeral 1.

. The medical device 1 under examination can be generally employed for anorectal inspections/operations, and comprises retractor means 2 adapted to be inserted within and to hold the anorectal canal patulous; inserting means 3 adapted to promote the insertion of said device 1 in said anorectal canal; and reversibly inflatable means 4 to apply in a controlled manner a pressure on the walls of said anorectal canal.

. The retractor means 2, essentially constituting an anoscope, comprise an essentially cylindrical hollow body 5 connected with handling means 6, through a joining member 7, all being manufactured preferably as a single piece. In particular, hollow body 5 and handling means 6 are jointed at an obtuse angle, so as to allow excellent ergonomics for the device. Again, in order to promote an ergonomic grip on the device, the outer surface of the handling means 6 has a corrugated progression on the side which is intended to contact the user's fingers.

. The hollow body 5 has a proximal end 8 connected to said joining portion 7 and a free distal end 9.

. The distal end 9 has preferably a bevelled edge. The proximal end 8 has a flared shape towards the outer side, and the joining member 7 with the handling means 6 extends from a portion thereof, as stated above.

. A graduated scale 26 is arranged on the hollow body 5 inner or outer surface.

. The handling means 6 have an essentially cylindrical shape. Preferably, said handling means 6 are hollow and opened at both ends. In particular, the end connected to the joining member 7 is provided with a hole 10 allowing the passage of lighting means, as will be described below.

. The joining member 7 directly connects the hollow body 5 proximal end 8 with the handling means 6. Supporting means 11 for said lighting means (not shown) are arranged on said joining member 7, essentially aligned with the handling means 6 adjacent opening. Preferably, such supporting means 11 are represented by two flexible tabs 12 defining a space in which said lighting means are inserted. Preferably, the lighting means can be represented by an optical bar as the one described in the Italian patent No. 1,234,169 by the same Applicant.

. At least the retractor means 2 hollow body 5 is made of transparent or translucent material, so as to be able to examine the walls of the anorectal canal arranged at the outer part.

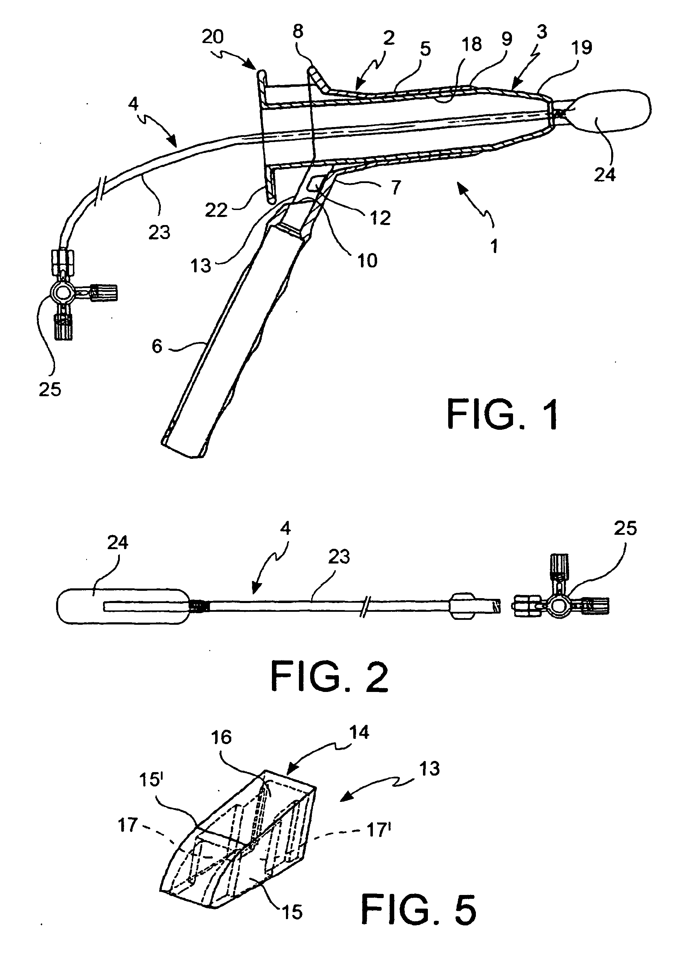

. Shielding means 13 for the light emitted by the lighting means are removably arranged on the joining member 7 open side facing the operator. Such shielding means 13, shown in Fig. 5, comprise a hollow member 14 composed by two side walls 15, 15' of a shape assimilable to a right-angled triangle, and by an upper wall 16 joining the side walls 15, 15' along the essentially oblique side thereof. Therefore, the hollow member 14 other two faces result to be open. The side walls 15, 15' inner surface has grooves 17, 17' which can be coupled to said tabs 12.

. The inserting means 3 comprise a hollow core 18, which is usually called mandrel or introducer, of an essentially cylindrical shape. The core 18 comprises a tapered distal end 19 - so as to promote the insertion in the anorectal canal - ending with a hole 21, and a proximal end 20 which is opened and ending with a radially outwardly projecting flange 22. Between flange 22 and core 18 surface, one or more tongues 30 extend which have a profile essentially complementary to the one of the flared proximal end 8 inner surface of the hollow body 5. Such tongues 30 constitute spacing means of the inserting means 3 relative to the retractor means 2.

. The inserting means 3 have such a shape and dimensions so as to be reversibly insertable in said retractor means 2, and have such a length so as to project from the retractor means 2 distal end, thus making at least the whole tapered portion to emerge.

. The reversibly inflatable means 4, shown in detail in Fig. 2, comprise a catheter 23, a reversibly inflatable member 24 being connected at an end thereof, a balloon in the example given. Both catheter 23 and balloon 24 are made of plastic/elastomeric materials compatible with a medical use. Advantageously, the catheter 23 end may be partially inserted within the balloon 24, the collar of which will be tied to the catheter 23 body.

. At the opposite end, the catheter 23 is connected to valving means 25 of a conventional type for a manual use. In particular, the valving means can be composed of a manually actuatable two-way valve, so as to be able to select an inlet passageway for air in the inflatable member, and an outlet passageway for air from said inflatable member.

. The assembling and functioning of the medical device 1 of the present invention will be now described.

. The medical device 1 can be assembled in an easy and safe manner by inserting the inserting means 3 within the retractor means 2 hollow body 5 until the tongues 30 abut against the retractor means 2 proximal end 8, and inserting the reversibly inflatable means 4 within the inserting means 3 core 18, thus making the balloon 24 projecting outside the hole 21. The lighting means are introduced from the opening of the handling means 6 up to the supporting means 11, therefore inserting them between the tabs 12. Then, the shielding means 13 are arranged astride of the lighting means, in engagement with the tabs 12.

. The use of the medical device 1 according to the invention firstly provides for the insertion thereof, according to the conventional art, in the patient's anorectal canal. Now, the device of the invention allows performing four discrete assessments, described herein below.

. The first assessment is the Rectal Sensitivity Test (TSR) which, in turn, allows the detection of three important diagnostic parameters:

. - First Sensation (first sensation of the contents of the rectal ampulla)

. - DDV (amount of air which is necessary to detect the sustained defecatory desire)

. - MTV (Maximum Tolerated Volume) beyond which the patient experiences pain.

. These three parameters are assessed by inserting, for example by means of a syringe or other pumping means, a measured amount of air through the valving means 25, so as to inflate the balloon 24, and detecting the sensation experienced by the patient as a function of the amount of air input. Preferably, both inserting means 3 and retractor means 2 are withdrawn so as not to interfere with the patient's sensations. The valving means 25 also act as stop means for the means 2, 3, which, therefore, will remain attached to the catheter 23 in performing the above-mentioned tests. At the end of the test, the balloon 24 is deflated through the valving means 25. Modifications of the above-mentioned parameters compared to those of an healthy patient represent important indicators of anorectal diseases.

. The second assessment which can be performed with the medical device 1 is the so-called Balloon Expulsion Test (BET). This test consists in inflating, as previously described, the balloon 24, placed in the rectum, with 60 cc air and requesting the patient to expel it within 1 minute. Again in this case, the test will be preferably performed with means 2, 3 withdrawn from the anorectal canal. Expulsion times above one minute are indicative of possible anorectal diseases.

. The third assessment which can be performed with the medical device 1 according to the invention is the assessment of the extent of the rectal prolapse (VEP). This assessment is very important for the presurgery preparation of the mucosal prolapse of the rectum, thus providing a selection of the most suitable surgery. The VEP measurement is obtained by properly placing the catheter in the patient's rectum and inflating the balloon 24 with 150-160 cc of air. The patient is then requested to expel the balloon 24, and at the same time the practitioner tends to withdraw it by operating on the catheter 23. In this manner, the balloon 24 presses on the rectal wall, and tends to withdraw the rectal mucosa as a sheath. The amount of mucosa protruding from the anus is assessed, both qualitatively and quantitatively, through the scaling which is present on the retractor means 2 hollow body 5 (in the latter case, the test shall be performed with the means 2 inserted).

. The fourth assessment which can be performed with the medical device 1 is the measurement of the anal canal length, a parameter which is important in the choice of preset surgeries. This measurement becomes possible thanks to the graduated scale provided on the retractor means 2 hollow body 5 and to the transparency of the walls of the same. In practice, after the device 1 has been inserted in the anal canal, and inserting means 3 and reversibly inflatable means 4 have been withdrawn, the anal canal length can be directly observed and measured.

. Each of the above-cited assessments can be performed by the practitioner independently one from the other, in the same manner as the device of the invention can be used simply as a conventional anoscope, according to the needs.

. From what has been reported above, the advantages of the medical device 1 of the invention shall be immediately understood. In fact, such device, beside the routinary application, makes a series of assessments possible, which assessments could be performed in the past only with the aid of complex and expensive equipments, and surely not concomitantly with the first proctological examination.

. The early assessment of the above-mentioned parameters provides the practitioner the possibility to diagnose the disease at the best, and to advise the patient -with full knowledge of the facts- to the most suitable pharmacological or surgical therapy.

. The medical device 1 is disposable, therefore it is characterized by extreme hygiene and ease of use.

. The constructive simplicity of the device of the invention makes it cost-effective and reliable.

. It shall be apparent that those skilled in the art will be able to make a number of variations to the instrument, without anyhow departing from the protection scope defined by the claims which are annexed herein.

## Claims

1. A medical device (1) comprising handling means (6) and retractor means (2) comprising an essentially cylindrical hollow body (5), which body has a proximal end (8) being connected to said handling means (6) through a joining member (7), supporting means (11) for lighting means being arranged on said joining member (7), essentially aligned with the adjagent opening of the handling means (6), said supporting means (11) being represented by two flexible tabs (12) defining a space in which said lighting means are inserted, said hollow body further comprising handling means (6) adapted to be inserted and to keep a patient's anorectal canal patulous, and inserting means (3) adapted to promote the insertion of said device (1) in said anorectal canal, said medical device (1) further comprising reversibly inflatable means (4) in order to apply a pressure on the walls of said anorectal canal in a controlled manner, said medical device (1) **characterized in that** shielding means (13) for the light emitted by said lighting means are removably arranged on the joining member (7) open side facing the operator and comprise a hollow member (14) composed of two side walls (15, 15') and by an upper wall (16) joining the side walls (15,15'), the inner surface of said side walls (15,15') having grooves (17,17') which can be coupled to said tabs (12).

2. The medical device (1) according to claim 1, wherein a graduated scale (26) is arranged on the hollow body (5) inner or outer surface.

3. The medical device (1) according to claim 2, said handling means (6) being hollow and opened at both ends in order to allow the passage of lighting means.

4. The medical device (1) according to any one of the claims 1 to 3, wherein said lighting means are an optical bar, and said supporting means (11) are represented by two flexible tabs (12) defining a space into which said optical bar is inserted.

5. The medical device (1) according to any one of the claims 2 to 4, wherein at least the hollow body (5) of the retractor means (2) is made of a transparent or translucent material, so as to be able to examine the anorectal canal walls which are arranged outside when the device (1) is being used.

6. The medical device (1) according to any one of the claims 1 to 5, said inserting means (3) comprising a hollow core (18) of an essentially cylindrical shape, said core (18) comprising a tapered distal end (19) ending with a hole (21), and a proximal end (20) which is opened and ending with a radially outwardly projecting flange (22).

7. The medical device (1) according to claim 6, wherein said inserting means (3) have such a shape and dimensions as to be reversibly insertable in said retractor means (2), and have such a length as to project from the retractor means (2) distal end, thus making at least the whole tapered portion emerge therefrom.

8. The medical device (1) according to any one of the claims 1 to 7, wherein said reversibly inflatable means (4) comprise a catheter (23), a reversibly inflatable member (24), such as a balloon for medical use, being connected to an end thereof.

9. The medical device (1) according to claim 8, said catheter (23) being connected, at the end which is free from said balloon (24), to valving means (25) adapted to select an inlet passageway for air into said reversibly inflatable member (24) and an outlet passageway for air from said reversibly inflatable member (24) outwardly.

10. The medical device (1) according to any one of the claims 1 to 9, wherein said reversibly inflatable means (4) are reversibly insertable in said inserting means (3), and wherein said reversibly inflatable member (24) is insertable in the hole (21) at the distal end (19) of said inserting means (3) and emerges outwardly.

## Patentansprüche

1. Medizinische Vorrichtung (1), die Griffmittel (6) und Rückziehmittel (2) aufweist, die mit einem wesentlichen zylindrischen Hohlkörper (5) verbunden sind, wobei der Körper ein proximales Ende (8) aufweist, das durch ein Verbindungselement (7) mit den Griffmitteln (6) verbunden ist, wobei Trägermittel (11) für Beleuchtungsmittel an dem Verbindungselement (7) angeordnet sind, im Wesentlichen ausgerichtet zu der benachbarten Öffnung der Griffmittel (6), wobei die Trägermittel (11) durch zwei flexible Zungen (12) repräsentiert sind, die einen Zwischenraum definieren, in den die Beleuchtungsmittel eingesetzt werden, wobei der Hohlkörper ferner Griffmittel (6) aufweist, zum Einsetzen und Aufhalten eines Anorektalkanals eines Patienten ausgelegt, und Einsetzmittel (3), die dazu ausgelegt sind, das Einsetzen der Vorrichtung (1) in den Anorektalkanal zu begünstigen, wobei die medizinische Vorrichtung (1) ferner reversibel aufpumpbare Mittel (4) aufweist, um auf die Wände des Anorektalkanals in kontrollierter Weise einen Druck auszuüben, wobei die medizinische Vorrichtung (1) **dadurch gekennzeichnet ist, dass** Abschirmmittel (13) für das von den Beleuchtungsmitteln abgegebene Licht an der zum Bediener weisenden offenen Seite des Verbindungselements (7) entfernbar angeordnet sind und ein hohles Element (14) aufweisen, das aus zwei Seitenwänden (15, 15') und einer oberen Wand (16), die die Seitenwände (15, 15') verbindet, zusammengesetzt ist, wobei die Innenoberfläche der Seitenwände (15, 15') Nuten (17, 17') aufweist, die mit den Zungen (12) gekoppelt werden können.

2. Die medizinische Vorrichtung (1) nach Anspruch 1, worin eine eingeteilte Skala (26) an der Innen- oder Außenoberfläche des Hohlkörpers (5) angeordnet ist.

3. Die medizinische Vorrichtung (1) nach Anspruch 2, worin die Griffmittel (6) hohl sind und an beiden Enden offen sind, um den Durchtritt von Beleuchtungsmitteln zu erlauben.

4. Die medizinische Vorrichtung (1) nach einem der Ansprüche 1 bis 3, worin die Beleuchtungsmittel ein optischer Stab sind, und die Trägermittel (11) durch zwei flexible Zungen (12) repräsentiert sind, die einen Zwischenraum definieren, in den der optische Stab eingesetzt wird.

5. Die medizinische Vorrichtung (1) nach einem der Ansprüche 2 bis 4, worin zumindest der Hohlkörper (5) der Rückziehmittel (2) aus transparentem oder durchscheinenden Material hergestellt ist, um die Anorektalkanalwände untersuchen zu können, die außerhalb angeordnet sind, wenn die Vorrichtung (1) benutzt wird.

6. Die medizinische Vorrichtung (1) nach einem der Ansprüche 1 bis 5, worin die Einsetzmittel (3) einen im Wesentlichen zylinderförmigen hohlen Kern (18) aufweisen, wobei der Kern (18) ein verjüngtes distales Ende (19) aufweist, das mit einem Loch (21) endet, sowie ein proximales Ende (20), das offen ist und mit einem radial nach außen vorstehenden Flansch (22) endet.

7. Die medizinische Vorrichtung (1) nach Anspruch 6, worin die Einsetzmittel (3) eine solche Form und solche Dimensionen haben, dass sie in die Rückziehmittel (2) reversibel einsetzbar sind, und eine derartige Länge haben, dass sie vom distalen Ende der Rückziehmittel (2) vorstehen, um zu bewirken, dass zumindest der gesamte verjüngte Abschnitt daraus austritt.

8. Die medizinische Vorrichtung (1) nach einem der Ansprüche 1 bis 7, worin die reversibel aufpumpbaren Mittel (4) einen Katheter (23) aufweisen, an dessen Ende ein reversible aufpumpbares Element (4), wie etwa ein Ballon zum medizinischen Gebrauch, angeschlossen ist.

9. Die medizinische Vorrichtung (1) nach Anspruch 8, worin der Katheter (23) an dem Ende, das von dem Ballon (24) frei ist, mit Ventilmitteln (25) verbunden ist, die dazu ausgelegt sind, einen Einlasskanal für Luft in das reversibel aufpumpbare Element (4) und einen Auslasskanal für Luft aus dem reversibel aufpumpbaren Element (24) nach außen auszuwählen.

10. Die medizinische Vorrichtung (1) nach einem der Ansprüche 1 bis 9, worin die reversibel aufpumpbaren Mittel (4) in die Einsetzmittel (3) reversibel einsetzbar sind, und worin das reversibel aufpumpbare Element (24) am distalen Ende (19) der Einsetzmittel (3) in das Loch (21) einsetzbar ist und davon vorsteht.

## Revendications

1. Dispositif médical (1) comprenant des moyens de manipulation (6) et des moyens formant écarteur (2) comprenant un corps creux essentiellement cylindrique (5), lequel corps a une extrémité proximale (8) qui est raccordée auxdits moyens de manipulation (6) par le biais d'un élément de joint (7), des moyens de support (11) pour des moyens d'éclairage qui sont agencés sur ledit élément de joint (7), essentiellement alignés avec l'ouverture adjacente des moyens de manipulation (6), lesdits moyens de support (11) étant représentés par deux languettes souples (12) définissant un espace dans lequel lesdits moyens d'éclairage sont insérés, ledit corps creux comprenant en outre des moyens de manipulation (6) adaptés pour être insérés et pour maintenir un canal anorectal d'un patient ouvert, et des moyens d'insertion (3) adaptés pour favoriser l'insertion dudit dispositif (1) dans ledit canal anorectal, ledit dispositif médical (1) comprenant en outre des moyens gonflables de manière réversible (4) afin d'appliquer une pression sur les parois dudit canal anorectal d'une manière contrôlée, ledit dispositif médical (1) étant **caractérisé en ce que** des moyens de protection (13) pour la lumière émise par lesdits moyens d'éclairage sont agencés de manière amovible sur le côté ouvert de l'élément de joint (7) faisant face à l'opérateur et comprennent un élément creux (14) composé de deux parois latérales (15, 15') et par une paroi supérieure (16) assemblant les parois latérales (15, 15'), la surface interne desdites parois latérales (15, 15') ayant des rainures (17, 17') qui peuvent être couplées auxdites languettes (12).

2. Dispositif médical (1) selon la revendication 1, dans lequel une échelle graduée (26) est agencée sur la surface interne ou externe du corps creux (5).

3. Dispositif médical (1) selon la revendication 2, lesdits moyens de manipulation (6) étant creux et ouverts aux deux extrémités afin de permettre le passage des moyens d'éclairage.

4. Dispositif médical (1) selon l'une quelconque des revendications 1 à 3, dans lequel lesdits moyens d'éclairage sont une barre optique et lesdits moyens de support (11) sont représentés par deux languettes souples (12) définissant un espace dans lequel ladite barre optique est insérée.

5. Dispositif médical (1) selon l'une quelconque des revendications 2 à 4, dans lequel au moins le corps creux (5) des moyens formant écarteur (2) est réalisé avec un matériau transparent ou translucide, afin de pouvoir examiner les parois du canal anorectal qui sont agencées à l'extérieur lorsque le dispositif (1) est utilisé.

6. Dispositif médical (1) selon l'une quelconque des revendications 1 à 5, lesdits moyens d'insertion (3) comprenant un noyau creux (18) d'une forme essentiellement cylindrique, ledit noyau (18) comprenant une extrémité distale progressivement rétrécie (19) se terminant par un trou (21) et une extrémité proximale (20) qui est ouverte et se terminant par un rebord (22) faisant saillie radialement vers l'extérieur.

7. Dispositif médical (1) selon la revendication 6, dans lequel lesdits moyens d'insertion (3) ont une forme et des dimensions telles qu'ils peuvent être insérés de manière réversible dans lesdits moyens formant écarteur (2) et ont une longueur telle qu'ils font saillie de l'extrémité distale des moyens formant écarteur (2), faisant ainsi sortir au moins toute la partie progressivement rétrécie.

8. Dispositif médical (1) selon l'une quelconque des revendications 1 à 7, dans lequel lesdits moyens gonflables de manière réversible (4) comprennent un cathéter (23), un élément gonflable de manière réversible (24) tel qu'un ballonnet à usage médical, qui est raccordé à son extrémité.

9. Dispositif médical (1) selon la revendication 8, ledit cathéter (23) étant raccordé, au niveau de l'extrémité qui est dépourvue dudit ballonnet (24), aux moyens de valve (25) adaptés pour sélectionner une voie de passage d'entrée pour l'air dans ledit élément gonflable de manière réversible (24) et une voie de passage de sortie pour l'air à partir dudit élément gonflable de manière réversible (24) vers l'extérieur.

10. Dispositif médical (1) selon l'une quelconque des revendications 1 à 9, dans lequel lesdits moyens gonflables de manière réversible (4) peuvent être insérés de manière réversible dans lesdits moyens d'insertion (3) et dans lequel ledit élément gonflable de manière réversible (24) peut être inséré dans le trou (21) au niveau de l'extrémité distale (19) desdits moyens d'insertion (3) et ressortir vers l'extérieur.
